# EUROPEAN PATENT APPLICATION

(11) **EP 1 312 668 A1**
(43) Date of publication of application: **21.05.2003**
(21) Application number: 01958469.7
(22) Date of filing: 24.08.2001
(51) Int. Cl.: C12N 5/00

(54) **STEM CELL CULTURE MEDIUM AND CULTURE METHOD BY USING THE SAME**

(30) Priority: 25.08.2000 JP 2000256431
(71) Applicant: Asahi Kasei Kabushiki Kaisha, Osaka-shi, Osaka 530-8205 (JP); The John P. Robarts Research Institute, London, Ontario N6A 5K8 (CA)
(72) Inventor: SAKANO, Seiji, Fuji-shi, Shizuoka 416-0939 (JP); BHATIA, Mick, London, Ontario N6G 4M8 (CA)
(74) Representative: Forstmeyer, Dietmar, Dr. rer. nat., Dipl.-Chem.
(86) International application number: JP0107261
(87) International publication number: WO02016556

(57) **Abstract**

Provision of a novel culture medium for stem cells and a culture method by using the same.

A culture medium including human Notch ligand protein as an effective ingredient and a method for growing human stem cells by using this culture medium. A growth factor may be used together with a human Notch ligand protein. By using the method of the present invention, the number or frequency of such human stem cells, such as these having SRC (Scid Repopulating Cells) activity, or as one characterized by the CD34 positive, CD38 negative and lineage antigens negative properties, can be amplified.

## Description

### TECHNICAL FIELD

The present invention relates to a culture medium for human stem cells and a culture method by using the same.

### BACKGROUND ART

There are many types of cells in human blood and lymph, each of which plays an important role. For example, red blood cells transfer oxygen, platelets have hemostatic actions, and white blood cells and lymphocytes prevent infections. These various types of cells are driven from hematopoietic stem cells in bone marrow. It has been discovered that hematopoietic stem cells are differentiated into various types of blood cells, osteoclasts and mast cells, stimulated by various types of cytokines and environmental factors in a living body. Cytokines, such as erythropoietin (EPO) for differentiation into red blood cells, granulocyte colony-stimulating factor (G-CSF) for differentiation into white blood cells, and thrombopoietin (TPO) for differentiation into megakaryocytes that are platelet-producing cells have been discovered. The former two of these cytokines have already been applied to clinical uses.

Bone marrow transplant that is performed as a therapeutic method for various types of hematopoietic disorderhemodyscrasia represents transplantation of hematopoietic stem cells. The method of using peripheral blood-derived or cord blood-derived hematopoietic stem cells has been adopted, and at present these methods are referred to as hematopoietic stem cell transplants. In these methods, cord blood-derived hematopoietic stem cell transplants are supposed to completely replace bone marrow transplant in the future, due to the burden placed on donors and the high quality of hematopoietic stem cells obtained.

However, as a result of cord blood-derived hematopoietic stem cell transplants into infant patients of over 500 cases, the period for recovery of normal hematopoietic reconstitution capability after transplantation exhibits a negative correlation with the number of transplanted cells and exhibits a positive correlation with body weight (Rubinstein P. et al., New England J. Med., 339, 1565-77, 1998). From this point, the application of cord blood-derived hematopoietic stem cell transplants in to human adults greatly increases the period of recovery, and the adults are also susceptible to an increased risk of infectious diseases and a long period of hospitalization. Therefore, if these cord blood-derived hematopoietic stem cells were able to be amplified by *in vitro* culture, it would be expected that the period of hospitalization would be shortened and that cord blood-derived hematopoietic stem cell transplants into adults could be performed more safely.

Moreover, in bone marrow transplantation procedure, ordinarily, from 500 ml to one liter of bone marrow is taken from normal donors. The donors are often volunteers and need to be put under general anesthesia and hospitalized for at least several days. Moreover, the donor may possibly be killed by an accident due to the anesthesia required by the procedure in the worst case scenario as very low possiblity. If it is possible that bone marrow may be taken with such a small amount, such as that used bone marrow examinations and the hematopoietic stem cells from the marrow can be amplified by culture, then both general anesthesia and hospitalization would become needless and this kind of accident would never occur. From these points of view, amplification of bone marrow-derived hematopoietic stem cells has merit.

Moreover, in order to apply gene therapy to blood cells, hematopoietic stem cells are needed to be cultured *in vitro*. And if amplification of hematopoietic stem cells can become possible, the number of gene-transferred hematopoietic stem cells can be increased and therefore it is expected that the frequency of recovery from the disease can be increased.

### DISCLOSURE OF THE INVENTION

The object of the present invention is to provide a novel culture medium of hematopoietic stem cells and a method of culturing hematopoietic stem cells by using the same to meet the above-mentioned need of the world.

Notch, which is discovered in *Drosophila,* is a receptor-type membrane protein relevant to differentiation control of neuron, and has been found in a wide spectrum of animal species regardless of the category of invertebrates or vertebrates. As for ligand molecules, which activate this Notch receptor and transmit a signal that represses differentiation of cells, two (*Drosophila* Delta and *Drosophila* Serrate as ligands of *Drosophila* Notch) have beendiscovered(Artavanis-Tsakonas et al., Science 285, 770-776, 1999).

Especially, regarding gene cloning of human molecules, four types of molecules have been reported as human Notch homologue and five types of molecules have been reported as human Notch ligand. In detail, as Delta-type ligand, human Delta-1 (Human DLL1 in another name. Hereafter, referred to as human Delta-1 in the present application) , human Delta-2 (Human DLL4 in another name. Hereafter, referred to as human Delta-2 in the present application) and human Delta-3 (Human DLL3 in another name. Hereafter, referred to as human Delta-3 in the present application) , and as Serrate-type ligand, human Jagged-1 (Human Serrate-1 in another name. Hereafter, referred to as human Jagged-1 in the present application ) and human Jagged-2 (Human Serrate-2 in another name. Hereafter, referred to as human Jagged-2 in the present application) have been reported by the end of June, 2000.

For human Delta-1, the typical publications are International Publication Number W097/19172 and Gray et al., Am. J. Pathol. 154, 785-795, 1999. For human Delta-2, the typical publications are International Publication Number W098/51799 and Shutter et al., Genes Dev. 14, 1313-1318, 2000. For human Delta-3, the typical publications are Japanese Patent Application Laid-open Publication No. 11-299493 and Bulman et al., Nature Genetics 24, 438-441, 2000. For human Jagged-1, the typical publications are International Publication Number W096/27610, International Publication Number W097/19172 and Oda et al., Genomics 43, 376-379, 1997. For human Jagged-2, typical publications are International Publication Number W098/02458 and Luo et al., Mol. Cell. Biol. 17, 6057-6067, 1997.

These Notch ligand molecules are known to repress differentiation of various types of cell lines. For example, it has been shown that rat Jagged-1, one of rat Notch ligands, has differentiation repressive action of muscle undifferentiated cell lines (Lindsell et al., Cell 80, 909-917, 1995).

On the other hand, regarding blood cells, the fact that these five types of human Notch ligands repress differentiation of blood cells has been shown in International Publication Number W097/19172, International Publication Number W098/02458, International Publication Number W098/51799, Japanese Patent Application Laid-open Publication No. 11-299493. In these publications, the actions to undifferentiated blood cells that form colonies and to undifferentiated blood cells measured by LTC-IC are shown, and also the use regarding the *in vitro* culture of hematopoietic stem cells are disclosed. Moreover, in these. publications, the methods for preparing human Notch ligand proteins are shown. However, the action of human Notch ligand on hematopoietic stem cells having hematopoietic reconstitution capability are inadequately discussed in these publications, and also, preferred culture conditions of hematopoietic stem cells having hematopoietic reconstitution capability utilizing human Notch ligands are not known.

And, as we described at the beginning of the description, when we consider a use in terms of clinical applications, regarding *in vitro* culture of hematopoietic stem cells, amplification of hematopoietic stem cells having hematopoietic reconstitution capability is desired. However, to perform these evaluations in a human living body is practically impossible. Therefore, as a typical evaluation method that demonstrates the effectiveness regarding an *in vitro* culture using Notch ligands, an evaluation by a xeno-transplantation
model, in which human hematopoietic stem cells are transplanted into immune-deficient mice or into sheep fetuses, is considered. From these facts, in the present invention, as a method for evaluating the action of human Notch ligand on hematopoietic stem cells having hematopoietic reconstitution capability, the evaluation method using immune-deficient mice, that is, the one using NOD-SCID mice, has been used.

Moreover, as another evaluation system for hematopoietic stem cells, the evaluation by the expression of cell surface protein of hematopoietic stem cells was performed. As a cell surface protein of human hematopoietic stem cells, CD34 antigen has been known, and it has been known that there is a particularly more undifferentiated surface antigen, which is characterized by CD34 antigen positive, CD38 antigen negative and lineage antigens negative (CD34⁺ CD38⁻ Lin⁻) properties. And also, it has been elucidated that there are hematopoietic stem cells having hematopoietic reconstitution capability, which is detected in NOD/SCID mice with high frequency only in the type of cells with CD34 antigen positive, CD38 antigen negative and lieage antigens negative properties, from past experiments using human cord blood derived- hematopoietic stem cells (Bhatia et al., Proc. Natl. Acad. Sci. U. S. A. 94, 5320-5325, 1997). Therefore, by investigating the amplification of the cells having this surface antigen, change in quality of hematopoietic stem cells having hematopoietic reconstitution capability can be investigated. From these facts, in the present invention, as the method for assessing hematopoietic stem cells having hematopoietic reconstitution capability of human Notch ligands, the assessing method, in which the number and frequency of cells in the cell group with CD34 antigen positive, CD38 antigen negative and lineage antigens negative properties were detected by flow cytometry, was also used for investigation. Thus, in the present invention, CD34 antigen negative andlineage antigens negative cells are referred to as CD34⁺CD38⁻ cells hereafter.

As a result of extensive studies by using these assessing methods, under the culture conditions using human Notch ligand, as a result of investigation by the method of using the above-mentioned NOD/SCID mice and also by the method of investigating the above-mentioned cell surface antigen, it has been elucidated that it is possible to amplify the hematopoietic stem cells having more hematopoietic reconstitution capability under the culture conditions using human Notch ligand as compared with the culture conditions without human Notch ligand, thereby completing the present invention.

The present invention relates to a culture medium comprising a human Notch ligand protein as an effective ingredient and having activities which cause cells including human stem cells to be cultured, thereby maintaining or amplifying the number or frequency of human stem cells.

Moreover, the present invention relates to a method of culturing human stem cells, comprising culturing cells including human stem cells using this culture medium under conditions in. which the human stem cells are in contact with the human Notch ligand protein, thereby maintaining or amplifying the number or frequency of the human stem cells.

As for the human stem cells used in the above-mentioned culture medium and the method of the present invention, there are human hematopoietic stem cells, SRC (ScidRepopulatingCells) activity positive human cells, human CD34 antigen positive, CD38 antigen negative cells, or the like. And also, as a human Notch ligand protein, at least one type of human Notch protein, which is selected from the group consisting of human Delta-1 protein, human Delta-2 protein, human Delta-3 protein, human Jagged-1 protein and human Jagged-2 protein, is used.

Moreover, a growth factor may be included in the culture medium, in which human stem cells are cultured. As a growth factor, there is at least one, which is selected from the group . consisting of a stem. cell factors (SCF), FLT-3 ligand (FLT-3L), interleukin 3 (IL-3), interleukin 6 (IL-6), granulocyte colony -stimulating factor (G-CSF), and fibronectin.

That is, the present invention relates to the following culture medium and the method of culturing human stem cells using the same.
(1) A culture medium comprising a human Notch ligand protein as an effective ingredient and having activities which cause cells including human stem cells to be cultured, thereby maintaining or amplifying the number or frequency of human stem cells.
(2) The culture medium according to the above-mentioned (1), further comprising a growth factor.
(3) The culture medium according to the above-mentioned (1) or (2), wherein human Notch ligand protein is at least one type of human Notch ligand protein selected from the group consisting of a human Delta-1 protein, human Delta-2 protein, human Delta-3 protein, human Jagged-1 protein and human Jagged-2 protein.
(4) The culture medium according to any one of the above-mentioned (1) to (3), wherein the human stem cells are human hematopoietic stem cells having hematopoietic reconstitution capability.
(5) The culture medium according to any one of the above-mentioned (1) to (3), wherein the human stem cells are at least one type of human stem cells selected from the group consisting of SRC (Scid Repopulating Cells) activity positive cells and human CD34 antigen positive, CD38 antigen negative, lineage antigens negative cells.
(6) The culture medium according to any one of the above-mentioned (2) to (5), wherein growth factor is at least one selected from the group consisting of a stem cell factor (SCF), Flt-3 ligand (FLT-3L), interleukin 3 (IL-3), interleukin 6 (IL-6), granulocyte colony-stimulating factor (G-CSF), and fibronectin.
(7) A method of culturing human stem cells, comprising culturing cells including human stem cells using the culture medium according to any one of the above-mentioned (1) to (6) under conditions in which the human stem cells are in contact with the human Notch ligand protein, thereby maintaining or amplifying the number or frequency of the human stem cells.
(8) The method according to the above-mentioned (7), wherein the human stem cells are human hematopoietic stem cells having hematopoietic reconstitution capability.
(9) The method according to the above-mentioned (7), wherein' human stem cells are at least one type of human stem cells selected from the group consisting of SRC (Scid Repopulating Cells) activity positive cells and human CD34 antigen positive, CD38 antigen negative, lineage antigens negative cells.

The details of the present invention are explained as follows.

A series of molecular biological experiments including preparation of cDNA needed in gene manipulations, examination of gene expression by Northern blot technique, screening by hybridization, preparation of recombinant DNA, determination of DNA nucleotide sequence, preparation of a cDNA library, or the like, can be performed by methods described in conventional laboratory manuals. As above-mentioned conventional laboratory manuals, for example, "Molecular Cloning, A laboratory manual, 1989, Eds., Sambrook, J., Fritsch, E. F. and Maniatis, T., Cold Spring Harbor Laboratory Press" can be utilized.

A "Human stem cell" as described in the present invention is a cell derived from human fetal tissues or human adult tissues, and is defined as differentiable cells into several types of cell lines, wherein human neural, stem cells, human mesenchymal stem cells, human hematopoietic stem cells and human hepatic stem cells, or the like, are included. Moreover, "human hematopoietic stem cells" are derived from human fetal tissues or human adult tissues, and defined as differeritiable cells into every type of blood cells, which constitute blood. Human hematopoietic stem cells having hematopoietic reconstitution capability are included in these "human hematopoietic stem cells." And also, "SRC (Scid Repopulating Cells) activity positive human cells", which are defined for these human stem cells more functionally, are defined as human stem cells detected by the methods described in the examples of the present invention, in "Bhatia et al., Proc. Natl. Acad. Sci. U. S. A. 94, 5320-5325, 1997; Dick et al., Stem Cells 15 Suppl. 1, 199-203; 204-207, 1997" or in "Eaves et al., Ann. NY Acad. Sci. 872, 1-8, 1999". And, moreover, "human CD34⁺ CD38⁻ cells", which is defined for these human stem cells by concretely using cell membrane proteins, are human stem cells measured by using commercially available antibodies by such a method as shown in the examples of the present invention. And also, "hemopoietic progenitor cells" described in the present invention is a general term for a blood cell group, which is destined to differentiate into specific blood cells that can be identified by using the methods shown in the examples of the present invention, that is, a blood colony assay or the like.

Moreover, a growth factor as described in the present invention is a general term for factors that enhance cell proliferation. As to forms of a growth factor, regardless of forms of factors such as a free protein or a cell membrane protein, a growth factor means a factor, of which any kind of action that enhances cell proliferation is described in scientific literature or the like. As a growth factor, for example, a stem cell factor (SCF), Flt-3 ligand (FLT-3L), interleukin 3 (IL-3), interleukin 6 (IL-6), granulocyte colony-stimulating factor (G-CSF), or the like, may be referred to. Moreover, in the present invention, the two groups of molecules, which ordinarily have general terms of adhesion molecule and extracellular matrix molecule respectively, are also supposed to be growth factors in a wide sense, and are then also included as growth factors, since functions of these molecules are related to cell proliferation. For example, fibronectin is a typical growth factor.

And also, in the present invention, human Notch ligand protein is a general term for proteins that transmit what is called a Notch signal through at least four types of Notch receptors (Notch 1 to 4), which have been found in mammals.

More concretely stating, human Delta-1, human Delta-2, human Delta-3, human Jagged-1, human Jagged-2 or the like, including the molecules shown in the examples of the present invention, are included. Moreover, especially in the case of human Notch ligand protein, of which the molecular form is shown as a general term of extracellular protein (polypeptides), extracellular partial protein (polypeptides), DSL domain (polypeptides), or the like, described in International Publication Number W097/19172, International Publication Number W098/02458, International Publication Number W098/51799 and Japanese Patent Application Laid-Open Publication No. 11-299493, the human Notch ligand protein is defined as a protein that can cause transmission of Notch signal. In this case, transmission of a Notch signal means that some change in cells caused through a Notch signal receptor can be shown as a Notch signal.

Moreover, as a form of existence of Notch ligand protein, there is a structure having a polymer structure. For example, a chimeric protein of this Notch ligand protein with Fc portion of human IgG, which is used in the present invention, is a divalent protein, as described in International Publication Number W097/19172, International Publication Number W098/02458, International Publication Number W098/51799 and Japanese Patent Application Laid-Open Publication No. 11-299493. Thus, it is expected that the activity of molecules existing as multivalent molecules, that is, as polymers is generally stronger than that of molecules existing as monovalent proteins, that is, as monomers. As for the method, the method, wherein a Notch ligand protein is expressed as a chimeric protein with the Fc portion of human IgG, that is, a Notch ligand protein expressed as a polymer bound together through a disulfide-bond by the hinge portion of an antibody can be referred to. Also in addition, the method wherein a Notch ligand protein is expressed as a chimeric protein, in which the antibody recognition sites are expressed on the C-terminus andN-terminus sites, thereby forming a polymer by reacting the polypeptide including the extra-cellular portion of expressed human Notch ligand protein with an antibody that specifically recognizes antibody recognition sites on the C-terminus or N-terminus sites, or the like, can be referred to.

Moreover, a polymer-type human Notch ligand having high specific activity for a dimer or multimers, which consist of fusion proteins prepared to express the peptides having a form that causes disulfide bonds on the C-terminus, N-terminus or on other sites, can be obtained, by such methods. The activity of no other human Notch ligand proteins are affected, and a fusion protein of Notch ligand protein with only the hinge region of the antibody is expressed, thereby forming a dimer by disulfide bond, or the like.

And moreover, there are methods, wherein polymer structure is expressed by arranging one protein or more selected from the group of human Notch ligand proteins in series or in parallel by genetic engineering techniques, or the like. As to other methods, any type of method for providing Notch ligand protein with a polymer structure of a dimer or multimers, which are known at present, can be applied. Therefore, human Notch proteins having the form of a dimer or multimers prepared by genetic engineering techniques, are to be included in the present invention.

And also, as to other methods, a method for causing Notch ligand proteins to be polymerized by using chemical cross linking agents, can be referred. For example, dimethyl-suberimidate-dihydrochloride for crosslinking lysine residues, N-(γ-maleinimide-butyryloxy) succinimide for crosslinking thiol groups of cystein residue, glutaraldehyde for crosslinking amino groups, are referred to as crosslinking agents. Polymers of a monomer or a multimer can be formed by using these cross linking reactions. Therefore, human Notch ligandproteins having the form of polymers of a dimer or multimer, prepared by chemical cross linking agents, are to be included in the present invention.

In the present invention, culture medium means the general term of the components that are directly in contact with cells in culturing cells; concretely stating, a nutrient medium and culture container are included in culture medium. The culture carrier, consisting of cellulose or agarose added at the time of culturing cells, is also included. As to the forms of Notch ligand protein, the proteins having molecular forms described as above, may exist, in a case of dissolving inmedium for culturing cells, or in another case of.existing on a solid surface or the like. As to methods for fixing Notch ligand protein, there are several methods of adsorbing'electrostatically-charged proteins, of using the amino acid group and carboxyl group of the protein, and/or of using a proper spacer and of using cross linking agents used in the above-mentioned polymerization, for binding the protein to the culture container by covalent bonds.

As shown in the examples 4 and 5, the nutrient medium used in these examples can be referred to, as a culture medium exhibiting activity for maintaining or amplifying the number or frequency of human stem cells, by culturing cells including human Notch ligand protein as an effective ingredient and also including human stem cells. Moreover, human Notch ligand proteins having various types of forms can be used.

Regarding this effect, as shown in example 4, the number or frequency of human stem cells, characterized by human hematopoietic stem cell marker of CD34⁺ CD38⁻, can be amplified. And, as shown in example 5, the number or frequency of human stem cells detected as human stem cells by using a NOD/SCID mouse transplantation model, can be amplified.

In the present invention, since "the number or frequency" is provided, "the number" means an absolute number, and can be enumerated as the number. And also, "frequency" means frequency of existence in terms of probability, and is defined in terms of probability. For example, when human stem cells with the "frequency" of one tenth is changed into that with the "frequency" of one fifth, it means that the "frequency" is amplified, that is, the probability of existence is increased.

### BRIEF DESCRIPTION OF THE DRAWINGS

Figure 1 shows the results of an electrophoresis (SDS-PAGE) and a Western blot analysis (WB anti-IgG) of a human Delta-1 protein (hDelta-1 IgG) and a human Delta-2 protein (hDelta-2 IgG) in Example 1.
Figure 2 shows the results of an electrophoresis (SDS-PAGE) and a Western blot analysis (WB anti-IgG) of a human Jagged-1 protein (hJagged-1 IgG) in Example 1.
Figure 3 shows binding characteristics of human cord blood-derived cells of a human Delta-1 protein and a human Delta-2 protein in Example 3.
Figure 4 shows binding characteristics of human cord blood-derived cells of a human Jagged-1 protein in Example 3.
Figure 5 shows the change, that occurs with the passage of culture period of the total number of cells, of the total number of CD34⁺CD38⁻ cells, and of the total number of hemopoietic progenitor cells in the culture of CD34⁺CD38⁻ cells under the condition wherein human Delta-1 protein has been added to the culture as in Example 4.
Figure 6 shows the change with the passage of culture period of the total number of cells, of the total number of CD34⁺CD38⁻, and of the total number of hemopoietic progenitor cells in the culture of CD34⁺CD38⁻ cells under the conditions wherein human Delta-2 protein is added to the culture as in Example 4.
Figure 7 shows the change with the passage of culture period of the total number of cells, of the total number of CD34⁺CD38⁻, and of the total number of hemopoietic progenitor cells in the culture of CD34⁺CD38⁻ cells under the conditions wherein human Jaggedd-1 protein has been added to the culture as in Example 4.
Figure 8 shows the results of a Southern blot analysis of the chimerism of bone marrow cells of NOD/SCID mouse, into which the cells treated by a human Delta-1 protein are transplanted in Example 5.
Figure 9 shows the change with the passage of culture period of the chimerism of bone marrow cells of NOD/SCID mouse, into which the cells treated by a human Jagged-1 protein are transplanted in Example 5.
Figure 10 shows the results of a Southern blot analysis of the chimerism of bone marrow cells of NOD/SCID mouse, into which the cells treated by a human Jagged-1 protein are transplanted in Example 5.

### BEST MODE FOR CARRYING OUT THE INVENTION

The present invention will now be described in more detail by way of example. However, the present invention is not limited to the following examples.

### Example 1

### <Preparation of a human Notch ligand extracellular protein>

A fusion protein of the extracellular portion of a human Notch ligand with the Fc portion of a human IgG1 is prepared and purified, according to the method described in the International Publication Number WO97/19172 for a human Delta-1, the International Publication Number WO98/51799 for human Delta-2, and. the International Publication Number WO97/19172 for a human Jagged-1, respectively.

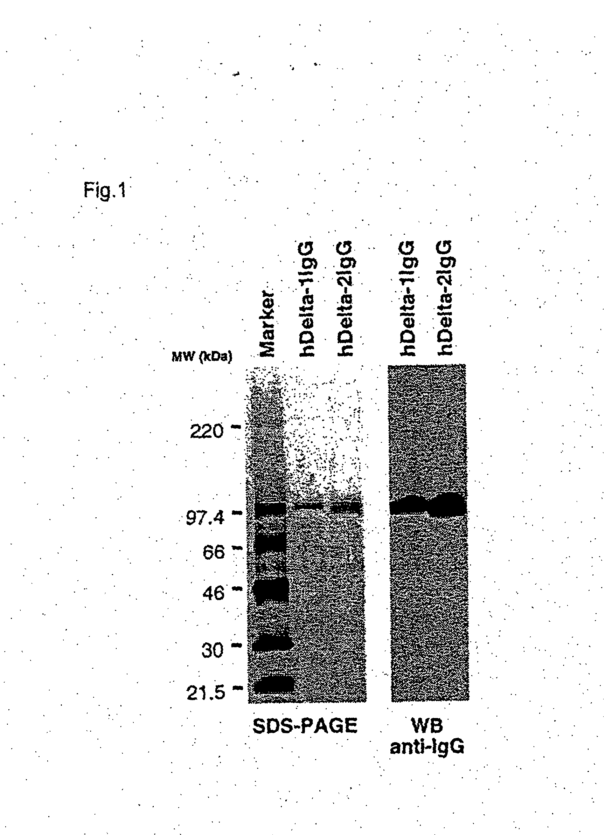

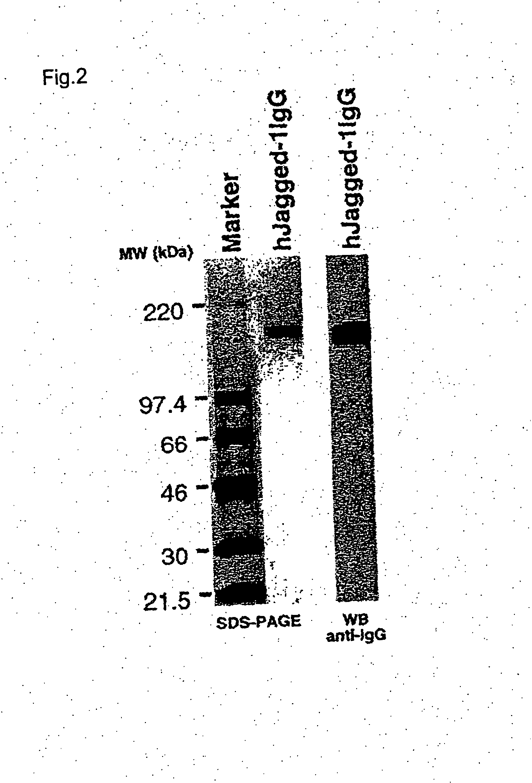

That is, each type of expression vector was prepared, according to the method described in the above-identified publications. They are expression vectors of a chimeric protein of human Delta-1 and human IgG Fc (hereafer, referred to as D1Fc), an expression vector of a chimeric protein of human Delta-1 and FLAG (hereafter, referred to as D1FLAG), an expression vector of a chimeric protein of human Delta-2 and human IgG Fc (hereafter, referred to as D2Fc), an expression vector of a chimeric protein of human Delta-2 and FLAG (hereafter, referred to as D2FLAG), an expression vector of a chimeric protein.of human Jagged-1 and human IgG Fc (hereafter, referred to as J1Fc), and an expression vector of a chimeric protein of human Jagged-1 and FLAG (hereafter, referred to as J1FLAG). Since these expression vectors have a neomycin resistance gene, gene-transferred cells were selected in a nutrient medium, which was added with neomycin. These expression vectors were gene -transferred into CHO cells by an electroporation method. These cells were cultured in a serum-free culture medium and supernatant was collected. The cells were concentrated and purified from the collected supernatant by column chromatography made of Protein A-Sepharose gel (Amarsham Pharmacia) or made of Sepharose gel immobilized with anti-FLAG antibody (Sigma). The ligand protein concentrated and purified like these was further purified by gel filtration, with buffer replaced with PBS (-)-buffer at the same time. Then the purified and separated human Notch ligand extracellular protein was obtained. The results of a SDS-PAGE of the chimeric proteins of these purified proteins with a human IgG and the results of a Western blot analysis with an anti-human Ig sheep antibody are shown in Figs.1 and 2. The markers used in the Figs. are Rainbow Markers made by Amersham-Pharmacia Biotech Co., Ltd.

### Example 2

### <Purification of cord blood-derived hematopoietic stem cells>

Human cord blood was diluted several times with α-MEM (Gibco BRL), the low-density cell fractions (< 1.077 g/ml) were fractionated by the method of specific gravity centrifugation using Ficoll Pack (Pharmacia, Sweden), and the cord blood mononuclear cells were separated. And also, lineage antigens negative cells (Lin⁻ cells) were concentrated by removing the cells that express differentiation antigen from these separated cord blood mononuclear cells, by the column method StepSep™ with magnetic beads, using Human Primitive Progenitor Enrichment Cocktail made by StemCell Technologies Co., Ltd. These Lin⁻ cells were stained with CD34 antibody (Becton Dickinson) labeled with Fluoresce in iso-thiocyanate (FITC) and with CD38 antibody (Becton Dickinson) labeled with allophycocyanin (APC), then a CD34⁺CD38⁻ cell was separated by the flow cytometer Vantage SE™ (Becton Dickinson). This cell is referred to as a CD34⁺CD38⁻ cell hereafter, and used in the following analyses.

### Example 3

### <Binding of Notch ligand extracellular protein to cord blood mononuclear cell>

A binding experiment of a Human Notch ligand extracellular protein prepared by the method disclosed in Example 1 to a cord blood mononuclear cell was performed. Then dilutions and washing of the solution used in the binding experiment were performed, by using PBS (-) solution (hereafter, dilution washing solution) including 25µg/ml calcium and 1% BSA (Sigma).

At the beginning, solutions used for reacting with cells, D1FLAG, D2FLAG or J1FLAG protein with the final concentration of 10µg/ml, anti-FLAG M2 antibody (Sigma) with the final concentration of 10 µg/ml and PE labeled anti-mouse IgG antibody (Becton Dickinson) with the dilution rate indicated in the attached manual, were prepared by diluting them with the following Blocking solution. Blocking solution was prepared, by preparing a dilution washing solution including a purified human IgG1κ (The Binding Site), with a concentration of 1 mM. The solution, with which cells react, had been prepared before reacting to cells, and was left in ice for reaction for one hour.

The cord blood mononuclear cells were added to the solution, with which the cells were prepared to be reacted, and were reacted in ice for one hour. Thereafter, a solution was added with dilution washing solution, was washed with a centrifuge, and was measure with the flow cytometer FACScalibur made by Becton Dickinson.

On the other hand, human IgG Fc chimeric protein of the human Notch proteins, that is, D1Fc D2Fc or J1Fc prepared in Example 1, were added to these experimental systems, and measurements were performed by the above-described methods. This measurement is performed in order to ensure that the binding of the above-described chimeric protein of a human Notch ligand protein and a FLAG to cord blood mononuclear cells is not nonspecific.

Moreover, in order to examine the contribution of a calcium ion to the binding, EDTA that chelates bivalent ions was added with the final concentration of 10 mM, and it was ensured that this binding was calcium dependent.

And also, the experiment was performed not only for a cord blood mononuclear cell as an object cell, but also for a LIN⁻ cell separated from the cord blood mononuclear cell as an object cell.

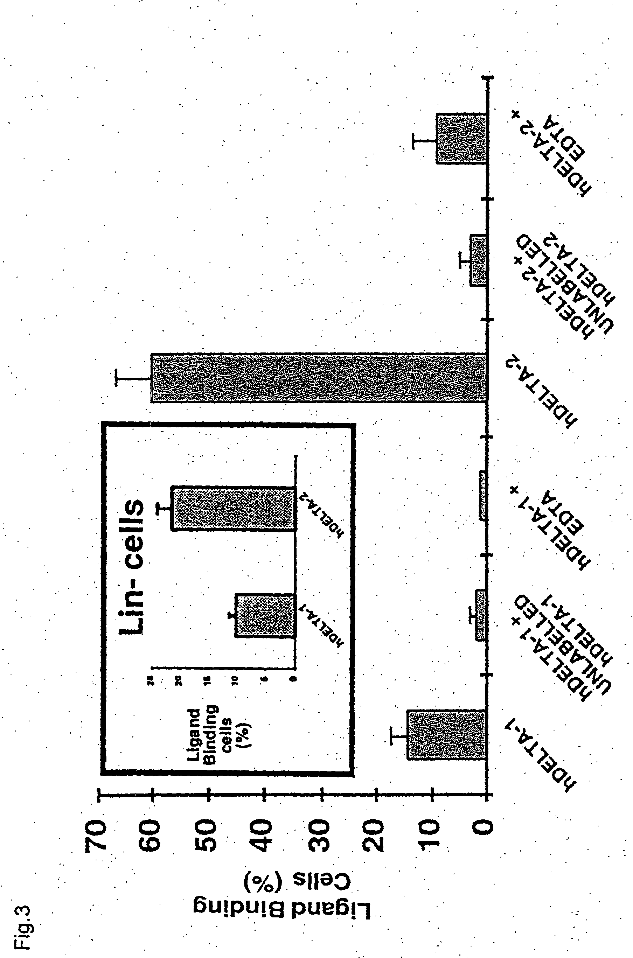

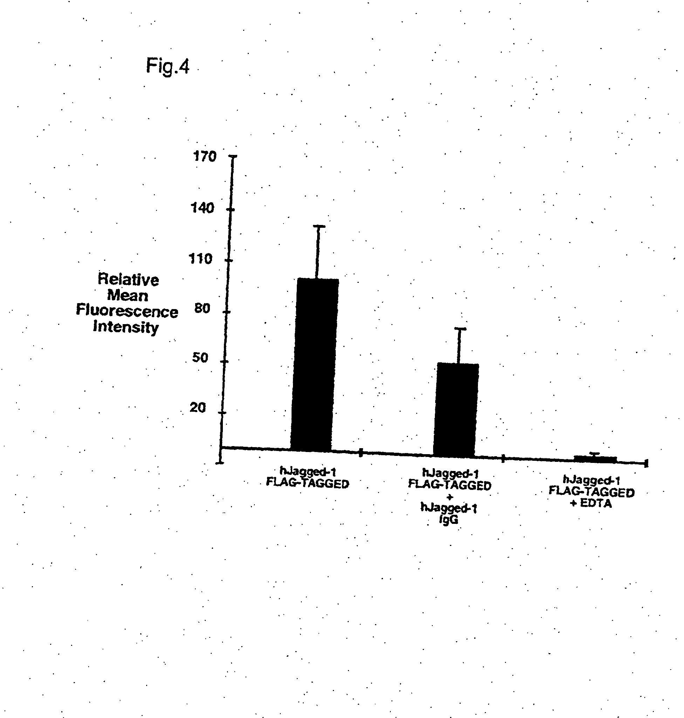

These results are shown in Fig.3 for human Delta-1 and human Delta-2, and in Fig.4 for human Jagged-1.

Human Delta-1 protein bound to about 15% of the cord blood mononuclear cells, and human Delta-2 protein bound to about 60% of the cells. And also, this binding was nearly completely inhibited with the addition of unlabeled ligands, that is, with addition of the chimeric protein of human Notch ligand protein and human IgG Fc. In the same way, the binding was inhibited with the addition of EDTA (Fig.3). And also, for Lin⁻ cells separated from a cord blood mononuclear cell, it was confirmed that a human Delta-1 cell was bound to about 10% of the cells and a human Delta-2 protein was bound to about 20% of the cells (the inner figure of Fig.3).

On the other hand, it was confirmed that the binding of a human Jagged-1 protein to Lin⁻ cells separated from cord blood mononuclear cells (Fig.4).

Still more, these resuls are shown, with the proportion of positive cells to non-stained cells in the control zone as the axis of ordinates in Fig.3, and are shown, with the mean fluorescence intensity as the axis of ordinates in Fig.4.

From these results, it is known that a human Notch ligand binds to a cord blood mononuclear cell, wherein binding to blood cells was observed and also it is known that a human Notch ligand binds to a Lin⁻ cell that a cord blood mononuclear cell does not express lineage antigens.

### Example 4

### <Effect of a human Notch ligand in culturing CD34⁺ CD38⁻ cells in a serum-free culture medium>

Human CD34⁺ CD38⁻ cells purified by the methods in Example 2 were cultured with a serum-free culture medium, the total number of cells, the number of CD34⁺ CD38⁻ cells and the number of hemppoietic progenitor cells were measured as time passed, under the conditions wherein human Notch ligand is added to the culture and without the addition of a human Notch ligand. CD34⁺ CD38⁻ cells were cultured by using 96-well plates (Becton Dickinson) with the initial number of cells of 500 to 2500 cells/well, under the conditions of 5% CO₂ at 37°C. Ninety six-well plates used in the culture had been previously coated with human fibronectin (Becton Dickinson), according to the methods of the attached. manual. And also, as a nutrient medium, Iscove's modified MEM nutrient medium (IMDM) including 20% BIT9500 (StemCell Technologies), which was added with 10⁻⁴ M β-mercaptoethanol and 2mM L-glutamine, was used. Still more, BIT9500 is the thing, in which BSA (Pre-buffered with NaHCO₃), bovine pancreas-derived insulin and iron-saturated human transferrin were dissolved in IMDMbase. And also, as growth factors, 300 ng/ml of human Flt3-L (R&D Systems), 10 ng/ml of human IL-3 (R&D Systems), 10 ng/ml of human IL-6 (R&D Systems), 300 ng/ml of human SCF (Amgen) and 50 ng/ml of human G-CSF (Amgen) were added to the nutrient medium.

For the addition of a Notch ligand, each of chimeric proteins, D1Fc, D2Fc and J1Fc, which were purified by the method of Example 1, was added separately to the nutrient medium at a concentration of 2 or 10 µg/ml, and the number of cells was compared between the culture with the addition of a Notch ligand and that without the addition of a Notch ligand.

The total number of cells after the culture was counted by using the counting method by microscope. And the total number of CD34⁺ CD38⁻ cells were calculated by measuring the proportion of CD34⁺ CD38⁻ cells by using the method shown in the Example 2, thereby multiplying the total number of cells by the proportion. And the number of hemopoietic progenitor cells was measured by using a colony formation method in a methylcellulose nutrient medium.

In a colony forming method, cells were cultured for 10 to 14 days, under the conditions with 5% CO₂ at 37°C, in a methylcellulose nutrient medium H4434 (StemCell Technologies) including 50 ng/ml human SCF, 10 ng/ml of human GM-CSF, 10 ng/ml of human IL-3 and 3 units/ml of human erythropoietin. The number of colonies for each type of cell was counted; a sharp discrimination was made between one type of colony and the other by using a microscope. The types of colonies were among a monocyte colony (CFU-M), a granulocyte colony (CFU-G), a granulocyte monocyte colony (CFU-GM), an erythroblast colony (BFU-E) and a combination colony (CFU-GEMM). And the total number of colony forming cells, that is, the total number of hemopoietic progenitor cells was calculated, by multiplying this value by the total number of cells.

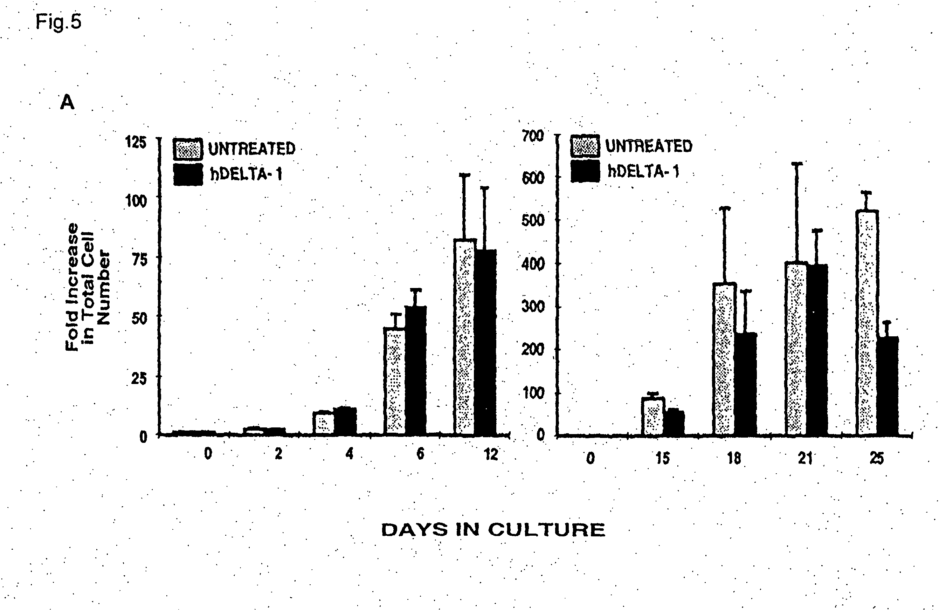

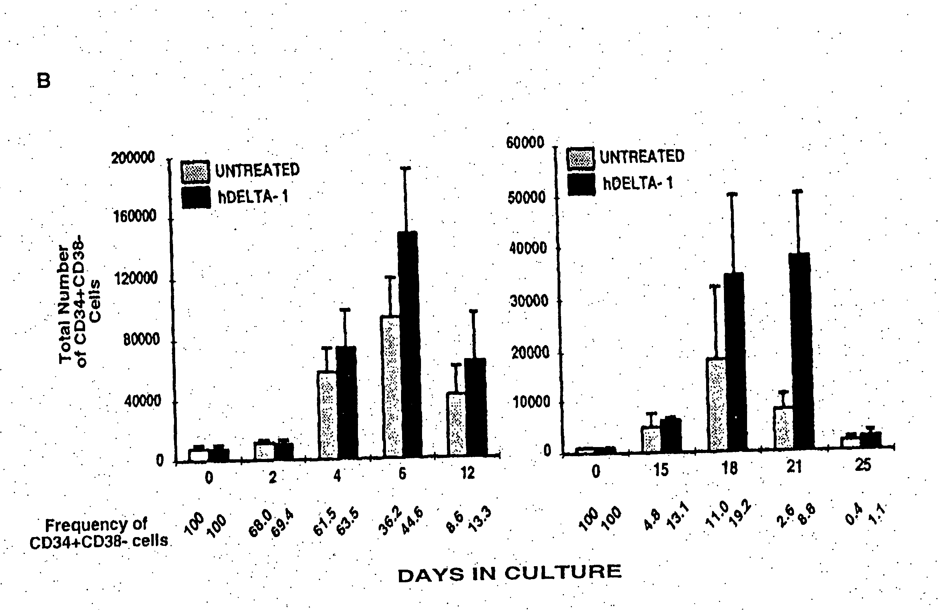

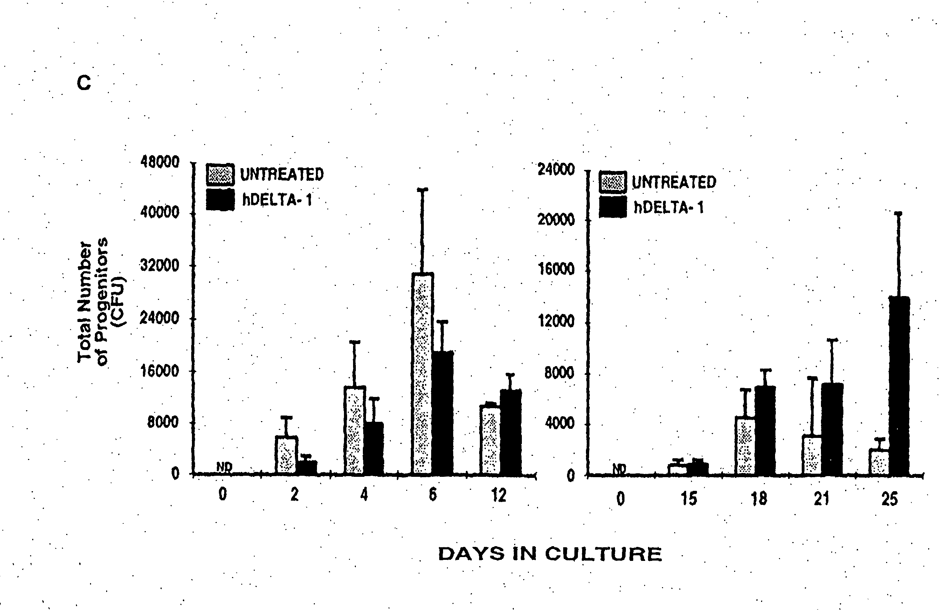

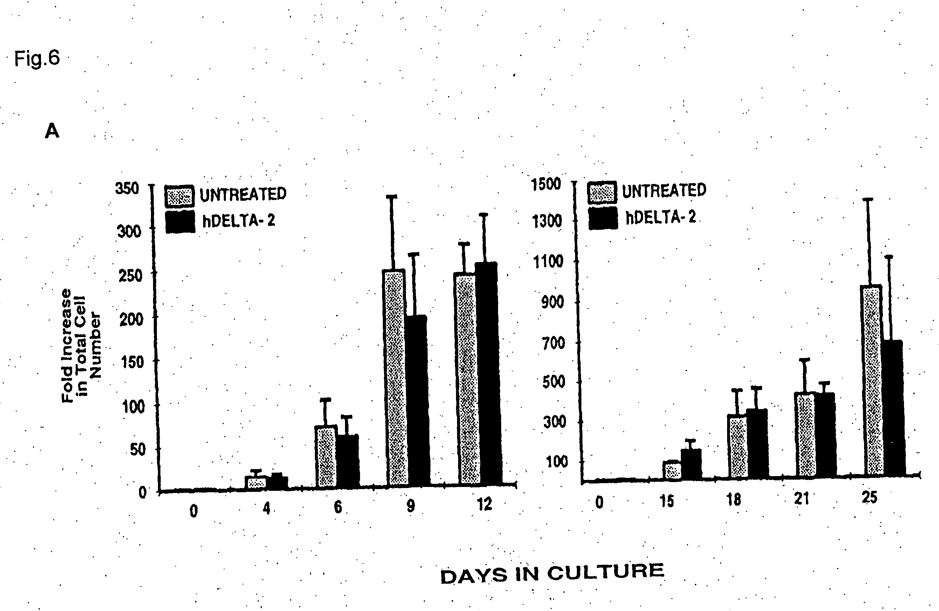

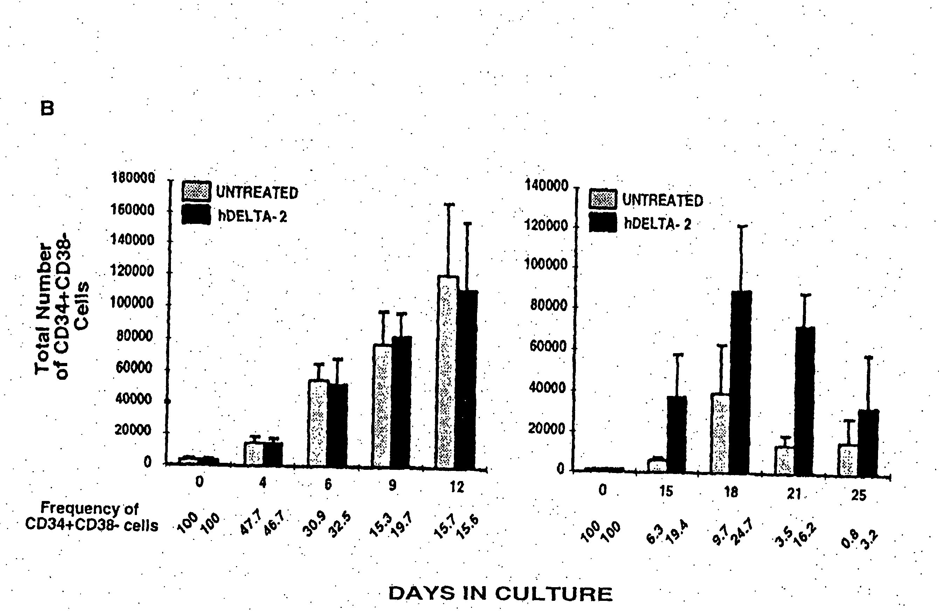

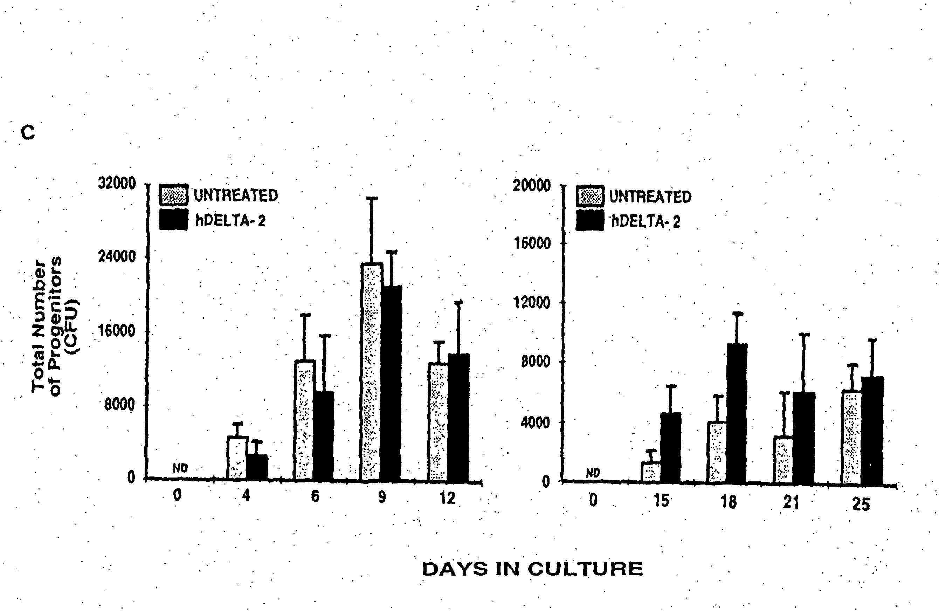

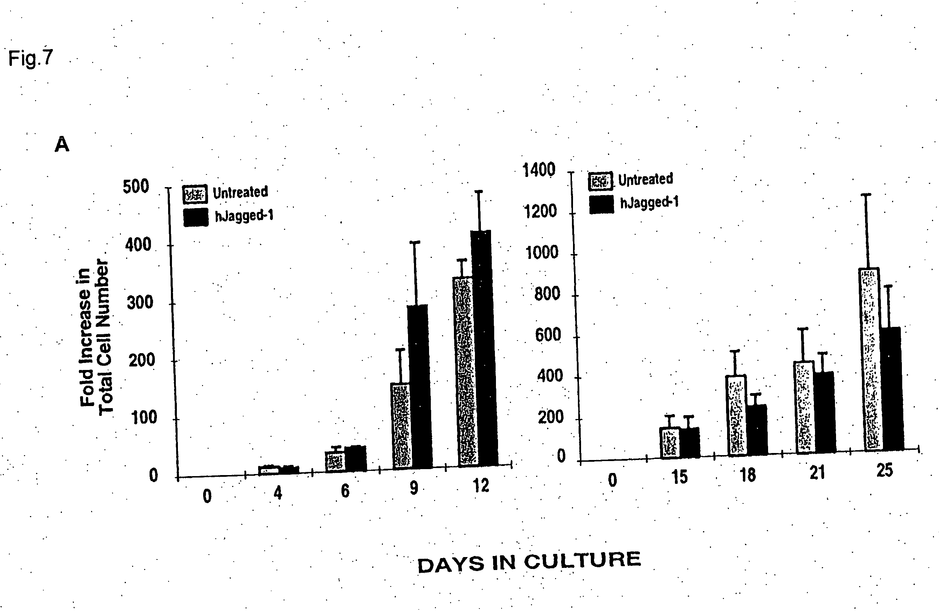

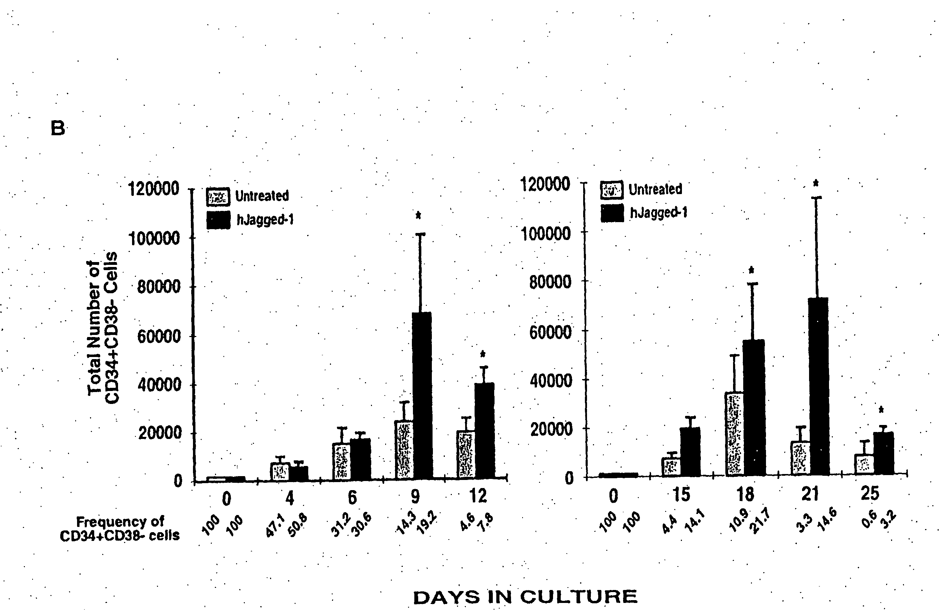

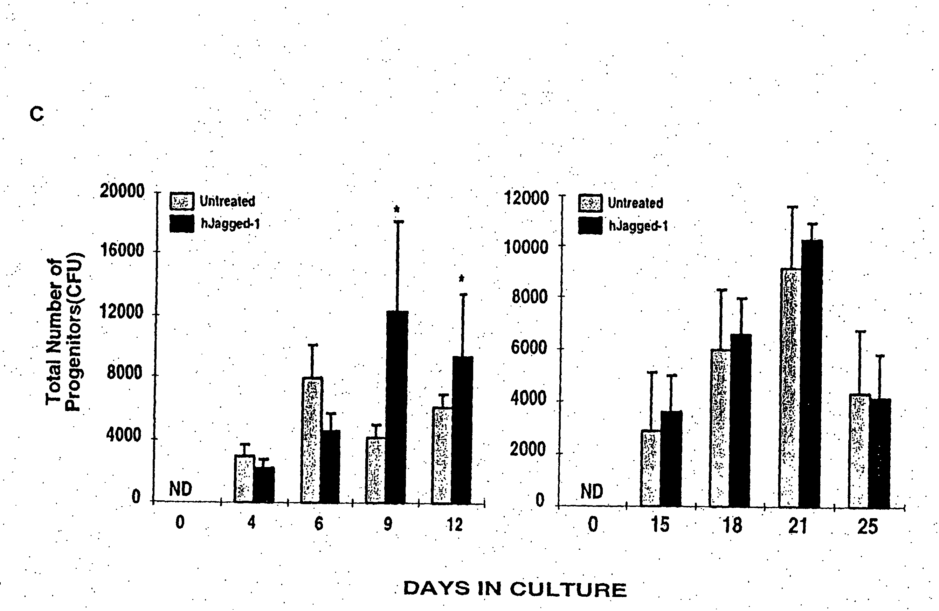

These results are shown in Fig.5 and Table 1 for human Delta-1, in Fig.6 and Table 2 for human Delta-2, and in Fig.7 and Table 3 for human Jagged-1.

As shown in Fig.5, no change was recognized particularly in the total number of cells, by the addition of human Delta-1 (Fig.5A). However, the total number of CD34⁺ CD38⁻ cells with the addition of human Delta-1 increased more than that without the addition of human Delta-1, and dramatically increased with the culture periods of 18 days and 21 days (Fig.5B). Moreover, it seemed that the total number of hemopoietic progenitor cells tended to decrease a little up to the 15^{th} day, however, the total number with the addition of human Delta-1 was dramatically larger than that without the addition of human Delta-1 with the culture period of 18 days or more (Fig.5C). And as shown in Table 1, with the details of. the types of colonies for these hemopoietic progenitor cells in mind, a tendency was recognized that though the proportion of CFU-M and CFU-G increased with a longer culture period without the addition of human Delta-1, the proportion of BFU-E increased with the addition of human Delta-1 on the contrary.

**Table 1**

| culture period (days) | addition | CFU-M (%) | CFU-G (%) | CFU-GM (%) | BFU-E (%) | CFU-GEMM (%) | seeding efficiency |
|---|---|---|---|---|---|---|---|
| 2 | no addition | 11.6 | 42.8 | 1.7 | 43.1 | 0.8 | 10.8± 6.8 |
| | h Delta-1 | 20.5 | 50.0 | 0 | 28 6 | 1.4 | 47.0± 27.9 |
| 4 | no addition | 14.6 | 44.6 | 1.2 | 39.1 | 0.7 | 12.8± 5.7 |
| | h Delta-1 | 11.5 | 41.0 | 0.8 | 46.9 | 0 | 18.3± 6.8 |
| 6 | no addition | 27.7 | 35.4 | 2.6 | 33.3 | 1.3 | 30.3± 20.3 |
| | h Delta-1 | 31.7 | 38.1 | 1.6 | 25 4 | 3.2 | 20.3± 1.9 |
| 12 | no addition | 34.4 | 20.0 | 5.1 | 30.8 | 9.7 | 32.0± 7.0 |
| | h Delta-1 | 24.1 | 26.5 | 1.6 | 43 5 | 4.3 | 26.5± 8.5 |
| 15 | no addition | 18.5 | 58.7 | 4.3 | 18.7 | 0 | 239.0+1 61.0 |
| | h Delta-1 | 7.8 | 50.6 | 0 | 41.7 | 0 | 67.5± 8.5 |
| 18 | no addition | 31.7 | 39.4 | 1.0 | 26.9 | 1.0 | 166.5±83.5 |
| | h Delta-1 | 3.9 | 14.1 | 0 | 79 9 | 2.1 | 43.0± 5.0 |
| 21 | no addition | 21.5 | 34.0 | 1.0 | 42.1 | 1.4 | 520.5±4 79.5 |
| | h Delta-1 | 11.1 | 20.2 | 0.4 | 66.7 | 1.6 | 140.0± 98.0 |
| 25 | no addition | 54.2 | 14.8 | 1.9 | 27.7 | 1.3 | 229.5± 48.5 |
| | h Delta-1 | 9.9 | 8.9 | 0.2 | 77.9 | 3.2 | 65.0± 3.0 |

And, as shown in Fig.6, no change was recognized particularly in the total number of cells, by the addition of human Delta-2 (Fig.6A). However, the total number of CD34⁺CD38⁻ cells dramatically increased with the culture periods of 15 days or more (Fig.6B). Moreover, it seemed that the total number of hemopoietic progenitor cells tended to decrease a little up to the 12^{th} day, however, it was confirmed that the total number with the addition of human Delta-2 tended to be larger than that without the addition of human Delta-2 with the culture period of 15 days or more (Fig.6C). And, as shown in Table 2,'with reading of the details of the types of colonies for these hemopoietic progenitor cells, no differences were recognized between the values with the addition of human Delta-2 and the values without the addition of human Delta-2, which is different from the case of the addition or non-addition of human Delta-1.

**Table 2**

| culture period (days) | addition | CFU-M (%) | CFU-G (%) | CFU-GM (%) | BFU-E (%) | CFU-GEMM (%) | seeding efficiency |
|---|---|---|---|---|---|---|---|
| 4 | no addition | 6.3 | 25.6 | 1.8 | 65.4 | 0.9 | 79.8± 73.4 |
| | h Delta-2 | 11.5 | 19.7 | 1.0 | 64 9 | 3.3 | 244.3± 218.9 |
| 6 | no addition | 11.7 | 23.6 | 1.1 | 50.6 | 13.0 | 18.0± 4.7 |
| | h Delta-2 | 12.5 | 21.6 | 1.1 | 64.0 | 0.8 | 38.7± 16.0 |
| 9 | no addition | 9.8 | 35.1 | 1.0 | 51.2 | 3.1 | 33.5± 10.6 |
| | h Delta-2 | 8.9 | 39.0 | 1.0 | 47 5 | 3.7 | 24.3± 4.5 |
| 12 | no addition | 12.7 | 33.0 | 8.7 | 32.0 | 13.5 | 52.0± 12.9 |
| | h Delta-2 | 11.6 | 43.6 | 1.3 | 42 3 | 1.3 | 121.7± 89.5 |
| 15 | no addition | 18.5 | 58.7 | 4.3 | 18.5 | 0 | 239.0± 161.0 |
| | h Delta-2 | 12.7 | 45.8 | 0.3 | 40.0 | 1.2 | 37.0± 4.0 |
| 18 | no addition | 21.7 | 38.1 | 0.7 | 39.1 | 0.3 | 86.0± 28.0 |
| | h Delta-2 | 12.5 | 37.1 | 0.4 | 49 4 | 0.6 | 40.0± 3.0 |
| 21 | no addition | 21.5 | 34.0 | 1.0 | 42.1 | 1.4 | 520.5± 479.5 |
| | h Delta-2 | 18.8 | 33.8 | 0.4 | 44.9 | 2.1 | 112.5± 57.5 |
| 25 | no addition | 19.4 | 25.3 | 13.4 | 36.0 | 5.9 | 175.0± 49.0 |
| | h Delta-2 | 17.1 | 25.5 | 0.5 | 55 6 | 1.4 | 99.0± 54.0 |

On the other hand, as shown in Fig.7, it is confirmed that the total number of cells was amplified with significance by the addition of human Jagged-1 at the 9^{th} day of culture period, however at other culture periods in particular, no differences were recognized in the total number of cells between the addition of human Jagged-1 and the nonaddition of human Jagged-1 (Fig.7A). And, it was also confirmed that the total number of CD34⁺CD38⁻ cells was dramatically amplified also at the 9^{th} day of culture period, and dramatic amplifications were also recognized at the longer culture periods, that is, at the 18^{th} day and at the 21th day (Fig.7B). In the same way, though it was confirmed that the total number of hemopoietic progenitor cells with the addition of human Jagged-1 tended to be larger than that without the addition of human Jagged-1 at the 9^{th} day of culture period, no differences were recognized between the numbers with the addition of human Jagged-1 and that without the addition of human Jagged-1 at the 15^{th} day of culture period or more (Fig.7C). And, as shown in Table 3, with the details of the types of colonies for these hemopoietic progenitor cells in mind, no differences were recognized between the values with the addition of human Jagged-1 and the values without the addition of human Jagged-1, different from the case of the addition or nonaddition of human Delta-1.

**Table 3**

| culture period (days) | addition | CFU-M (%) | CFU-G (%) | CFU-QM (%) | BFU-E (%) | CFU-GEMM (%) | seeding efficiency |
|---|---|---|---|---|---|---|---|
| 4 | no addition | 12.8 | 25.5 | 0.4 | 57.1 | 4.3 | 5.0± 1.2 |
| | h Jagged-1 | 12.8 | 28.5 | 0.5 | 55 6 | 2.8 | 6.0± 1.2 |
| 6 | no addition | 15.8 | 11.2 | 1.1 | 68.2 | 3.7 | 7.0± 1.0 |
| | h Jagged-1 | 13.9 | 13.2 | 1.0 | 69.2 | 2.3 | 16.0± 3.1 |
| 9 | no addition | 24.9 | 24.1 | 1.4 | 46.8 | 3.0 | 35.0± 2.5 |
| | h Jagged-1 | 16.2 | 20.7 | 0.4 | 59 | 2.8 | 44.5± 18.4 |
| 12 | no addition | 19.1 | 28.0 | 1.9 | 50.0 | 1.1 | 75.3± 8.3 |
| | h Jagged-1 | 14.9 | 43.5 | 1.8 | 39 5 | 0.4 | 110.0± 45.0 |
| 15 | no addition | 22.9 | 42.4 | 0.6 | 33.8 | 1.0 | 108.0± 59.0 |
| | h Jagged-1 | 12.9 | 49.8 | 0.2 | 35.8 | 1.3 | 55.3± 18.6 |
| 18 | no addition | 18.5 | 41.9 | 0.5 | 36.1 | 3.1 | 114.0± 53.4 |
| | h Jagged-1 | 14.0 | 34.1 | 0.7 | 47 7 | 3.4 | 38.3± 5.5 |
| 21 | no addition | 26.6 | 29.9 | 0.6 | 33.8 | 1.0 | 108.0± 59.0 |
| | h Jagged-1 | 12.9 | 49.8 | 0.4 | 41.1 | 2.1 | 73.0± 22.0 |
| 25 | no addition | 25.3 | 39.2 | 3.3 | 21.1 | 0.0 | 108.0± 59.0 |
| | h Jagged-1 | 31.7 | 41.6 | 2.4 | 30 2 | 2.2 | 130.0± 31.0 |

From these results, it has been elucidated that the addition of a human Notch ligand amplify the number of CD34⁺CD38⁻ cells.

### Example 5

### <The effects of human Notch ligand in the culture of CD34⁺CD38⁻ cells by the evaluation method using NOD/SCIDmousehematopoietic reconstitution capability >

The cells cultured by the method shown in Example 4 were transplanted to an immune-deficient mouse NOD/SCID, and about 60'days later, the existing rates of human cells were detected by Southern blot analysis and assessed.

In detail, 8-week-old NOD/LtSz-scid/scid (NOD/SCID) mice (purchased from The Jackson Laboratories, U.S.A., maintained and prepared in the John P. Robarts Research Institute) were labeled sublethally irradiated (355 cGy), human cord blood-derived CD34⁺CD38⁻ cells that were cultured under the conditions in Example 4 were transplanted to mice by tail-vein injection. The number of the cells transplanted to a mouse corresponds to the number of 500 to 2500 of CD34⁺CD38⁻ cells before culture. Moreover, according to the method of Bonnet et al. (Bone Marrow Transplant 23, 203-209, 1999), accessory cells of lineage antigens positive cells (LIN⁺) that were irradiated (1500 rads) at the same time were transplanted.

After 8 weeks passed after transplantation, bone marrow cells were taken out from the mice, and chromosomal DNA was separated by conventional methods, and was used for analyses. From one to two µg of the separated DNA was excised by the restriction enzyme EcoRI, was applied to agarose gel electrophoresis, and was analyzed according to the conventional methods of Southern blot analysis. A human chromosome 17-specific α-satellite probe (p12H8) (Lapidot et al. Science 255, 1134-1141, 1992) was used as a probe. A 2.7 kb band was detected on agarose gel electrophoresis, by using this probe. Still more, in order to investigate the proportion of chimerism, independently prepared mouse chromosomal DNA and human chromosomal DNA were mixed with a variety of proportions, and the mixture was treated in the same manner, and was analyzed by the methods of Southern blot analysis at the same time, for use as standard data of chimerism. By comparing the proportion of this standard chimerism with the density of the band for DNA samples on agarose gel electrophoresis, the proportion of chimerism was measured. The lower limit of detection is 0.05% of chimerism.

On the basis of chimerism data calculated by the above-described methods, the proportion of mouse, in which human cells were detected, were shown in Table 4, for human Delta-1 and human Delta-2. These results are shown as the proportion of the number of human cell positive NOD/SCID mice to the total number of transplanted mice. At four days of culture period, though nine mice out of 17 were human cell positive in the human Delta-1-treated zone, only four mice out of 17 were human cell positive in the human Delta-1-nontreated zone, showing the apparent increase in the transplantation efficiency of human cells by the human Delta-1 treatment. However, this difference was not recognized for 6 days of culture period. And, by the human Delta-2 treatment, no difference was recognized both for 4 days and for 6 days of culture period.

**Table 4**

| | 4 days of culture period | | | 6 days of culture period | | |
|---|---|---|---|---|---|---|
| | number of human cell positive | number of human cell negative | human cell implantation efficiency (number of positive/total number of implantation) % | number of human cell positive | number of human cell negative | human cell implantation efficiency (number of positive/total number of implantation) % |
| non-treatment | 4 | 13 | 4/17 (23%) | 4 | 5 | 4/9 (44%) |
| h Delta-1 | 9 | 8 | 9/17 * (53%) | 9 | 8 | 4/10 * (40%) |
| non-treatment | 2 | 10 | 2/12 (17%) | 1 | 5 | 1/6 (17%) |
| h Delta-2 | 2 | 10 | 2/12 (17%) | 0 | 5 | 0/5 * (0%) |

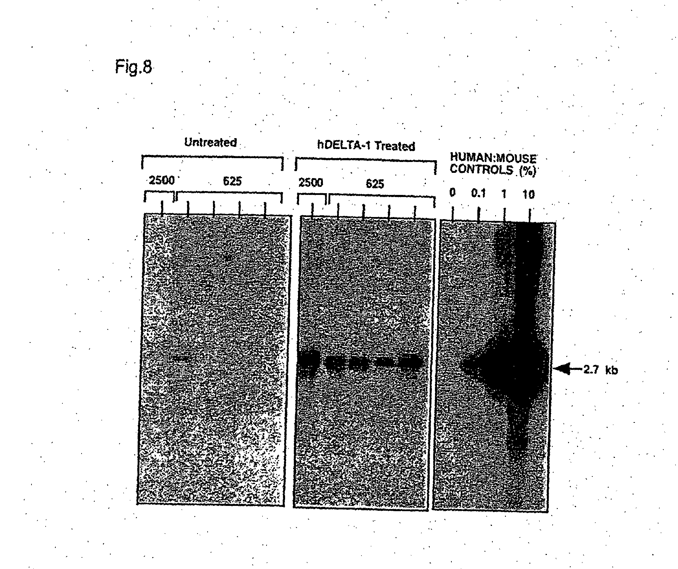

And, as another experiment, the results of experiment, in which the cells after the culture with the addition of human Delta-1 were diluted and transplanted to mice, were shown in Fig.8, as a data of Southern blot analysis. In this experiment, each of two sets of 2500 of CD34⁺CD38⁻ cells was cultured for 4 days with or without the addition of human Delta-1, and was divided into four portions in order to transplant to four mice, and then chimerism was examined. From the past studies, it has been elucidated that, in average, 2 of SRCs existed out of 2500 of CD34⁺CD38⁻ cells (Bhatia et al., J. Exp. Med., 186, 619-624, 1997). Therefore, ordinarily human cells were detected in two out of four mice, by using cells before culture. As a result, though human genes were detected in only one out of four mice without the addition of a human Delta-1, human genes were detected in all four out of four mice, showing successful transplantation.

From these results, it is apparent that the number or frequency of human stem cells detected by SRC increased, by culturing the cells with the nutrient medium including human Delta-1.

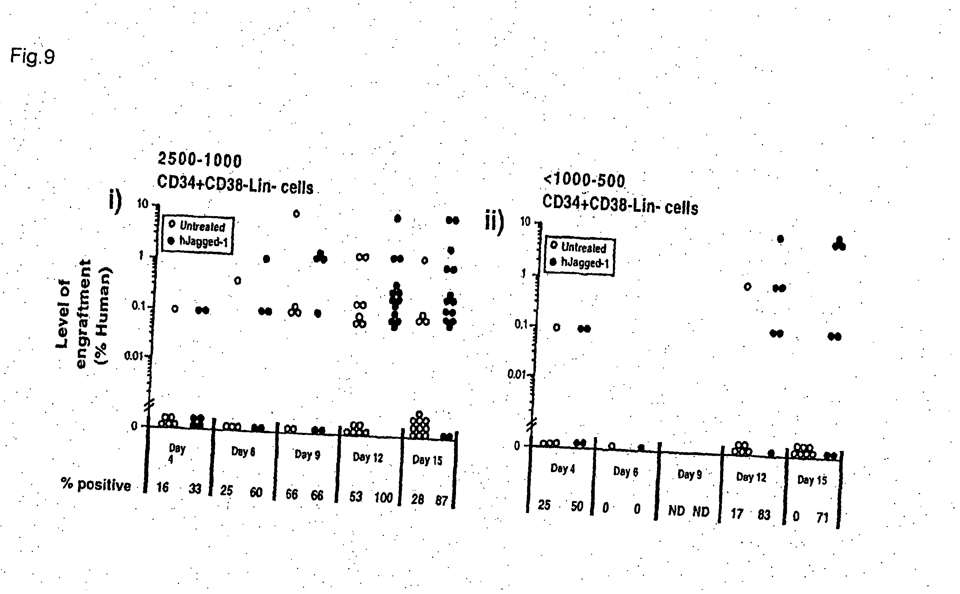

Next, a summary of data regarding human Jagged-1 calculated by the same methods is shown in Fig.9.

The figure (i) on the left-hand side is the results of the experiment, wherein pre-culture cells were 1000 to 2500 of cord blood-derived CD34⁺CD38⁻ cells, and the figure (ii) on the right-hand side is the results of the experiment, wherein pre-culture cells were 500 to 1000 of cord blood-derived CD34⁺CD38⁻ cells. The axis of ordinates shows the proportions of human chimerism (%), the axis of abscissas shows the days of culture period. And, the mark, o shows the data with only cytokines without the addition of human Jagged-1, the mark, • shows the data with the addition of human Jagged-1. And, the proportion (%), of which human genes were positive, were shown below for each result.

From the past studies, it has been elucidated that, in average, 2 of SRCs existed out of 2500 of CD34⁺CD38⁻ cells. Though, in the culture both for 4 days and 6 days, a big difference was not obtained in the proportion, of which human cells were detected, between the addition and the non-addition of human Jagged-1, however, in the culture for longer culture periods, for 12 days and 15 days, the proportion, of which human cells were detected, had dramatically increased by the addition of human Jagged-1. That is, in the case that 1000 to 2500 of the cells were transplanted, though the proportion was only 53% for 12 days of culture period and 28% for 15 days of culture period without the addition of human Jagged-1, the proportion, of which human cells were detected, was 100% for 12 days of culture period and 87% for 115 days of culture period with the addition of human Jagged-1. And, in the case that 500 to1000 of the cells were transplanted, though the proportion was only 17% for 12 days of culture period and 0% for 15 days of culture period without the addition of human Jagged-1, the proportion; of which human cells were detected, was 83% for 12 days of culture period and 71% for 15 days of culture period with the addition of human Jagged-1.

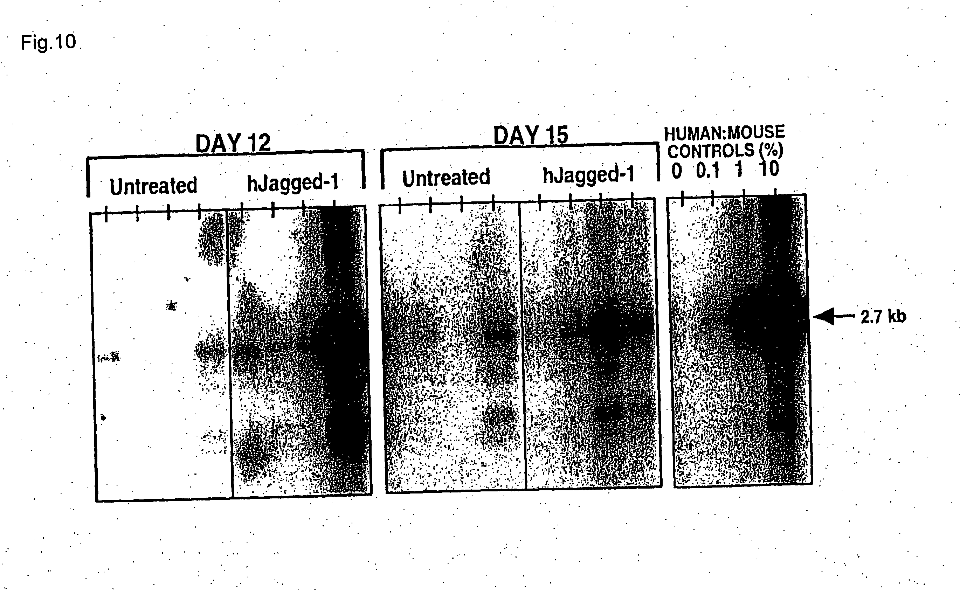

And, as an independent experiment, the results of an experiment, in which the cells after the culture with the addition of human Jagged-1 were diluted and transplanted to mice, are shown in Fig.10 as the result of Southern blot analysis. In this experiment, after 2500 of CD34⁺CD38⁻ cells were cultured for 12 days or 15 days with or without the addition of human Jagged-1, each culture was divided into four in order to transplant to 4 mice, and chimerism was examined. As described as above, from the past studies, it has been elucidated that, in average, 2 of SRCs existed out of 2500 cells. Therefore, human cells are detected in 2 out of 4 mice by using pre-culture cells ordinarily. As a result, in the culture for 12 days, though human genes were detected only in 2 out of 4 mice without the addition of human Jagged-1, human genes were detected in all 4 out of 4 mice with the addition of human Jagged-1. And, in the culture for 15 days, though human genes were detected only in 2 out of 4 mice without the addition of human Jagged-1, human genes were detected in 3 out of 4 mice with the addition of human Jagged-1. Moreover, the band density by Southern blotting analysis for the culture with the addition of human Jagged-1 was apparently stronger than that without the addition of human Jagged-1. From this fact, it is known that human cells appear more frequently in each of the mice, in which human cells were detected. From these results, it is apparent that the number or frequency of human stem cells detected by SRC increased by culturing cells in the nutrient medium including human Jagged-1.

And, human-derived cells in murine bone marrow transplanted with the cells cultured under the existence of any of these Notch ligands were stained with an antibody that recognizes various types of human antigens according to conventional methods, and were observed by using FACS. As a result, it was confirmed that all types of cells such as lymphoid cells (CD19, CD20), granulocytes (CD15, CD33), un-differentiated cells (CD34, CD38) were also produced with the addition of any one of various types of Notch ligands.

### INDUSTRIAL APPLICABILITY

By the present invention, the number or frequency of human stem sells having hematopoietic reconstitution capability can be amplified and the *in vitro* amplification of human stem cells becomes possible.

## Claims

1. A culture medium comprising a human Notch ligand protein as an effective ingredient and having activities which cause cells including human stem cells to be cultured, thereby maintaining or amplifying the number or frequency of the human stem cells.

2. The culture medium according to claim 1, further comprising a growth factor.

3. The culture medium according to claim 1 or 2, wherein the human Notch ligand protein is at least one type of human Notch ligand protein selected from the group consisting of a human Delta-1 protein, human Delta-2 protein, human Delta-3 protein, human Jagged-1 protein, and human Jagged-2 protein.

4. The culture medium according to any one of claims 1 to 3, wherein the human stem cells are human hematopoietic stem cells having hematopoietic reconstitution capability.

5. The culture medium according to any one of claims 1 to 3, wherein the human stem cells are at least one type of human stem cells selected from the group consisting of SRC (Scid Repopulating Cells) activity positive cells and human CD34 antigen positive, CD38 antigen negative, lineage antigens negative cells.

6. The culture medium according to any one of claims 2 to 5, wherein the growth factor is at least one selected from the group consisting of a stem cell factor (SCF), FLT-3 ligand (FLT-3L), interleukin 3 (IL-3), interleukin 6 (IL-6), granulocyte colony-stimulating factor (G-CSF), and fibronectin.

7. A method of culturing human stem cells, comprising culturing cells including human stem cells using the culture medium according to any one of claims 1 to 6 under conditions in which the human stem cells are in contact with the human Notch ligand protein, thereby maintaining or amplifying the number or frequency of the human stem cells.

8. The method according to claim 7, wherein the human stem cells are human hematopoietic stem cells having hematopoietic reconstitution capability.

9. The method according to claim 7, wherein human stem. cells are at least one type of human stem cells selected from the group consisting of SRC (Scid Repopulating Cells) activity positive cells and human CD34 antigen positive, CD38 antigen negative, lineage antigens negative cells.
